# EUROPEAN PATENT SPECIFICATION

(11) **EP 4 514 229 B1**
(45) Date of publication and mention of the grant of the patent: **17.12.2025**
(21) Application number: 23721607.2
(22) Date of filing: 20.04.2023
(51) Int. Cl.: A61B 8/06, A61B 5/00, A61B 5/08, A61B 5/083, A61B 5/145, A61B 8/08, A61B 8/00, A61H 31/00

(54) **ULTRASOUND VELOCITY/FLOW MEASUREMENTS FOR CPR FEEDBACK**
ULTRASCHALL-GESCHWINDIGKEITS-/FLUSSMESSUNGEN FÜR CPR-RÜCKKOPPLUNG
MESURES DE VITESSE/DÉBIT PAR ULTRASONS POUR RETOUR D'INFORMATIONS DE RCP

(30) Priority: 25.04.2022 US 202263334224 P; 30.06.2022 US 202263357156 P
(43) Date of publication of application: 05.03.2025
(73) Proprietor: Koninklijke Philips N.V., 5656 AG Eindhoven (NL)
(72) Inventor: JORGENSON, Dawn Blilie, 5656 AG Eindhoven (NL); CHEN, Shujie, 5656 AG Eindhoven (NL); PITTS, Conner David, 5656 AG Eindhoven (NL); GALLAGHER, Madison Kathleen, 5656 AG Eindhoven (NL); RICHARD, Christian James, 5656AG Eindhoven (NL)
(74) Representative: Philips Intellectual Property & Standards
(86) International application number: PCT/EP2023/060209
(87) International publication number: WO 2023/208699

(56) References cited:
- KR-A- 20180 031 991
- US-A1- 2010 022 886
- US-A1- 2010 076 315
- US-A1- 2020 107 989
- US-A1- 2021 275 131

## Description

### FIELD OF THE INVENTION

The present disclosure generally relates to ultrasound velocity/flow measurements for cardiopulmonary resuscitation ("CPR") feedback, and more particularly to devices, systems and methods that use ultrasound to acquire blood velocity or flow data which is then processed to provide audio and visual feedback without an ultrasound image.

### BACKGROUND OF THE INVENTION

The field of resuscitation is heavily focused on increasing CPR quality. An ultrasound sensor has been utilized for a non-invasive, reliable, and physiological CPR feedback by providing user-friendly indicators of blood velocity or flow. CPR responders may not be familiar with interpreting ultrasound images, so the velocity/flow data needs to be represented in a way that is easily understood by resuscitation professionals and lay people. More particularly, ultrasound usage is currently limited to those with ultrasound expertise. Certain medical fields, such as resuscitation, have vast opportunity for ultrasound devices, but most users are not trained to use traditional ultrasound devices.. For example, document US 2020/0107989 A1 discloses a blood flow sensor device such as a non-invasive cardiac arrest monitor that uses ultrasound to detect blood flow is used to monitor blood flow during cardiopulmonary resuscitation. One or more gating signal generation devices transmit gating signals to a blood flow monitoring computing device. The blood flow monitoring computing device uses the gating signals to determine time periods during which blood flow information generated by the blood flow sensor device is most likely to be accurate. The blood flow monitoring computing device presents the measured blood flow to a user.

### SUMMARY OF THE INVENTION

The present disclosure is directed to facilitating a non-sonographer (i.e. resuscitation responder) use of ultrasound to measure blood velocity/flow in a vessel without needing to interpret an image. The non-sonographer simply apply the ultrasound sensor correctly, then perform CPR with audio and visual indications of blood velocity/flow providing information to improve CPR quality. The ultrasound sensor can also detect a spontaneous pulse, and the audio/visual indicators relay that information. Manual palpation is currently the most widely used technique to determine the presence of a spontaneous pulse, but this is not as effective as using Doppler ultrasound. The ultrasound sensor can measure blood velocity or flow, and the same sensor will have LEDs or other indicators to easily convey these measurements. A medical device like a monitor/defibrillator can be integrated with the ultrasound sensor, and that medical device can also have audio/visual indicators to convey the same information. This information may be saved to a report at the end of a CPR event.

The present disclosure may be embodied as:
(1) a CPR feedback system;
(2) a CPR feedback controller; and
(3) a CPR feedback method.

Various CPR feedback system exemplary embodiments of the present disclosure encompass an ultrasound sensor configured to measure carotid blood velocity of the patient during CPR of the patient, and one or both of an oxygenation saturation sensor configured to measure blood oxygenation of the patient during CPR of the patient and a respiratory sensor configured to measure CO2 production of the patient during CPR of the patient. The various CPR feedback system embodiments of the present disclosure further encompass a CPR feedback controller configured to generate CPR feedback based on a descriptive correlation and/or a prescriptive correlation of a carotid blood velocity measurement by the ultrasound sensor and one or both of a blood oxygenation measurement by the oxygenation saturation sensor and a CO2 production measurement by a respiratory sensor.

For purposes of describing and claiming the present disclosure, the term "descriptive correlation" that broadly encompasses a relationship between the various measurements that is descriptive of an ongoing baseline CPR of a patient as will be exemplary described in the present disclosure, and the term "prescriptive correlation" a relationship between the various measurements that is prescriptive of a patient-specific CPR of a patient as will be exemplary described in the present disclosure.

Various CPR feedback controller exemplary embodiments of the present disclosure encompass a non-transitory machine-readable storage medium encoded with instructions for execution by one or more processors. The non-transitory machine-readable storage medium includes instructions to (1) receive a carotid blood velocity of a patient during CPR of the patient, (2) receive a blood oxygenation measurement of the patient and/or a CO2 production measurement of the patient during CPR of the patient, and (3) generate CPR feedback based on one of a descriptive correlation or a prescriptive correlation of a carotid blood velocity measurement and one or both of the blood oxygenation measurement and the CO2 production measurement.

Various CPR feedback methods exemplary embodiments of the present disclosure encompass (1) measuring, by an ultrasound sensor, a carotid blood velocity of a patient during CPR of the patient, (2) measuring, by an oxygenation saturation sensor, a blood oxygenation of a patient during CPR of the patient, and/or measuring, by a respiratory sensor, a CO2 production of a patient during CPR of the patient, and (3) generating, by a CPR feedback controller, CPR feedback based on one of a descriptive correlation or a prescriptive correlation of a carotid blood velocity measurement by the ultrasound sensor and at least one of a blood oxygenation measurement by the oxygenation saturation sensor and a CO2 production measurement by the respiratory sensor.

The foregoing exemplary embodiments and other exemplary embodiments of the present disclosure as well as various structures and advantages of the present disclosure will become further apparent to one having ordinary skill in the art from the following detailed description of various exemplary embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates an exemplary embodiment of a CPR feedback system in accordance with the present disclosure;
FIG. 2 illustrates an exemplary embodiment of a flowchart representative of a CPR feedback method in accordance with the present disclosure;
FIGS. 3-5 illustrate exemplary CPR feedback in accordance with the present disclosure;
FIG. 6 illustrates an exemplary identification of endotracheal tube placement in accordance with the present disclosure;
FIG. 7 illustrates an exemplary identification of carotid artery blockage in accordance with the present disclosure; and
FIG. 8 illustrates an exemplary embodiment of a CPR feedback controller in accordance with the present disclosure.

### DETAILED DESCRIPTION OF THE PREFERRED EMBODIMENTS

The present disclosure provides devices, systems and methods that will indicate the velocity or flow of blood in a vessel with (1) audio feedback via the ultrasound sensor itself and/or an integrated medical device (e.g. monitor/defibrillator) and (2) visual feedback (LEDs, infographic) via the ultrasound sensor itself and/or the integrated medical device. Exemplary uses include to measure blood velocity or flow in the carotid, femoral artery, or other vessel(s) to provide feedback during CPR, and a secondary use can be to indicate a patient's spontaneous pulse.

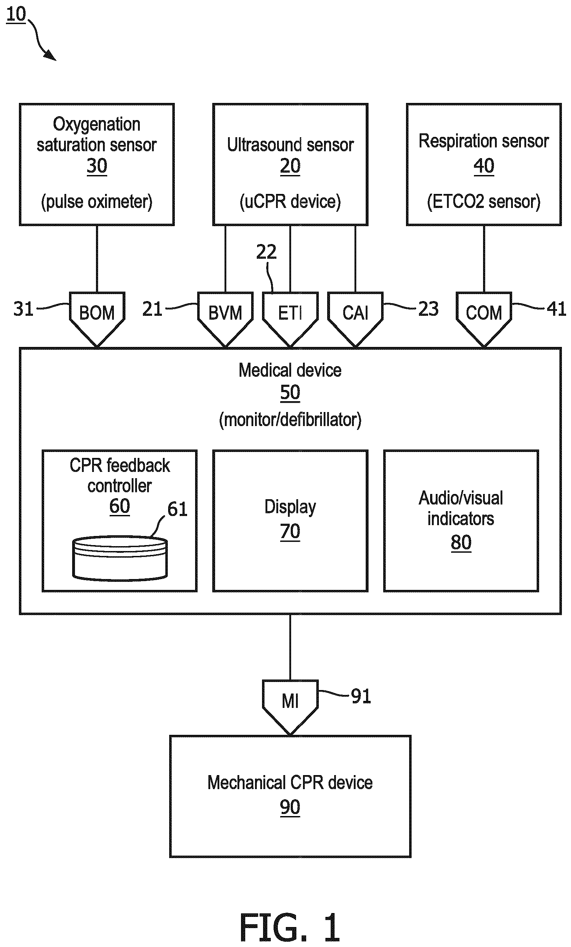

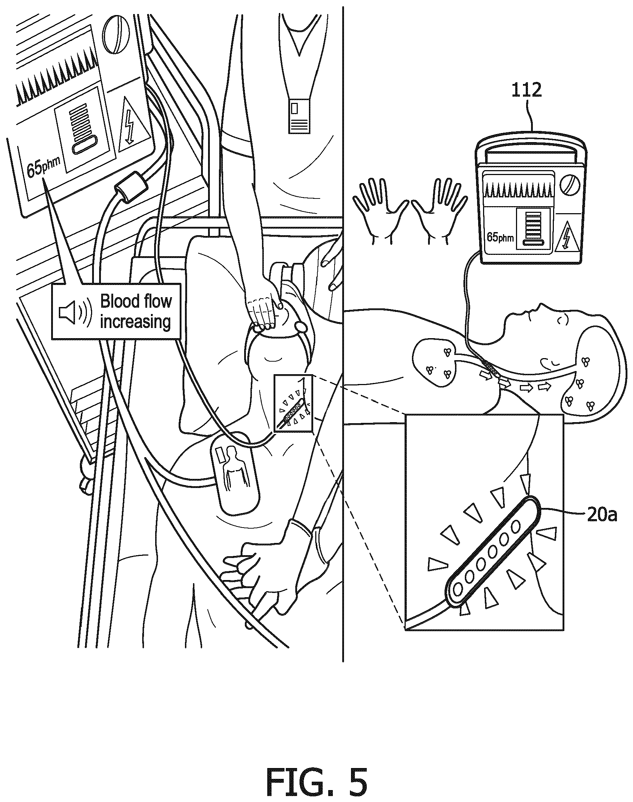

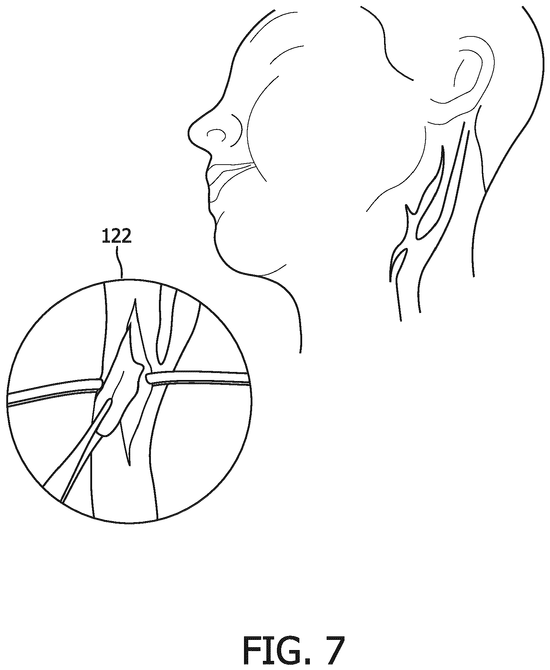

To facilitate an understanding of the present disclosure, the following description of FIGS. 1-7 teaches exemplary embodiments of CPR feedback devices, systems and methods in accordance with the present disclosure. From the description of FIGS. 1-7, those having ordinary skill in the art of the present disclosure will appreciate how to apply the present disclosure to make and use additional embodiments of CPR feedback devices, systems and methods in accordance with the present disclosure.

Referring to FIG. 1, a CPR feedback system 10 of the present disclosure employs an ultrasound sensor 20 (e.g., uCPR device), an oxygenation saturation sensor 30 (e.g., a pulse oximeter), a respiratory sensor 40 (concurrent with or alternative to sensor 30) (e.g., an ETCO2 sensor), a medical device 50 (e.g., a monitor/defibrillator) and a mechanical CPR device 90.

For purposes of describing and claiming the present disclosure, the term "ultrasound sensor" broadly encompasses any device, as known in the art of the present disclosure or hereinafter conceived, for generating ultrasound images illustrative of a blood velocity of a patient during CPR of the patient; the term "oxygenation saturation sensor" broadly encompasses any sensor, as known in the art of the present disclosure or hereinafter conceived, for directly or indirectly measuring a blood oxygenation of a patient during CPR of the patient; and the term "respiratory sensor" broadly encompasses any sensor, as known in the art of the present disclosure or hereinafter conceived, for directly or indirectly measuring a CO2 production by a patient during CPR of the patient, and the terms "medical device" and "mechanical CPR device" are terms of the art of the present disclosure.

Still referring to FIG. 1, medical device 50 includes a CPR feedback controller 60, a display 70 and audio/visual indicators 80.

For purposes of describing and claiming the present disclosure, the term "CPR feedback controller" broadly encompasses any type of controller, as known in the art of the present disclosure or hereinafter conceived, for controlling feedback of the CPR procedure to a user via a display and/or audio/visual indicators, and to a mechanical CPR device; and the terms "display" and "audio/visual indicators" are terms of the art of the present disclosure.

In practice, CPR feedback controller 60 can include a database 61 of specifying CPR guidelines, as known in the art of the present disclosure, such as, for example, compression rate, depth and duty cycle.

Still referring to FIG. 1, in accordance with certain exemplary embodiments of the present disclosure, the ultrasound sensor 20 applies Doppler ultrasound principles to measure blood velocity or flow 21. These measurements are processed by a CPR feedback controller 60 of device 50 and communicated via audio and/or visual indicators 80 of device 50. These measurements can also be processed by controller 60 to detect a spontaneous pulse in the patient, which can then also be relayed by audio and/or visual indicators 80.

In practice, the following phase teaches how to build certain exemplary embodiments of the present disclosure:
Phase 1 - Optimize ultrasound sensor 20 to get the best velocity/flow data for a particular vessel.
Phase 2 - Develop a user interface (UI) and a physical sensor design.
Phase 3 - Develop algorithm for translating ultrasound velocity/flow data to audio/visual feedback.
Phase 4 - Integrate the ultrasound sensor 20 with medical device 50. If this device has a monitor, develop UI and algorithm for translating ultrasound velocity/flow data to audio/visual data.
Phase 5 - Clinically verify ultrasound accuracy and device usability.
Phase 6 - Iterate through the design process as necessary to fine-tune the UI and sensor design for the intended users.

In practice, an exemplary method for using / use of the ultrasound sensor 20 includes (1) connecting the ultrasound sensor 20 to the medical device 50, (2) placing the ultrasound sensor on a patient's skin over a desired vessel (e.g., carotid artery), (3) the ultrasound sensor 20 measuring blood flow/velocity using Doppler and (4) the medical device 50 reporting these measurements to the user via audio and/or visual indicators 80. The user can easily interpret these measurements and change CPR technique if necessary as prompted or otherwise indicated or instructed to do so based on the audio and/or visual indicators.

The ultrasound sensor 20 uses the Doppler algorithm to detect the presence of a spontaneous pulse. When detected, the indicator(s) 80 show(s)/tell(s)/indicate(s) that a spontaneous pulse is detected and issues instructions to further guide the user accordingly.

Exemplary Applications of certain exemplary embodiments of the present disclosure include integrating ultrasound sensor 20 with Advanced Life Support (ALS) monitor/defibrillator products, for example. It can also be applicable to any compatible ultrasound system/sensor where ultrasound velocity/flow data need to be represented by infographics or other indicators rather than an ultrasound image, for example. Exemplary embodiments of the present disclosure can span virtually all clinical applications of ultrasound where fluid velocity/flow is measured.

As one having ordinary skill in the art should appreciate in view of the present disclosure and teachings herein, while blood flow is an important indicator of perfusion to the brain, the oxygen content of the delivered blood is also important. For example, if the patient has not been well ventilated or has an underlying condition impacting oxygenation (e.g., CO2 poisoning or hyperventilation or chronic respiratory disease), then perfusing the brain with blood that has diminished O2 will likely not be adequate for effective CPR and successful resuscitation of the patient.

Thus in addition to ultrasound sensor 20, system 10 and method disclosed and described herein above, certain exemplary embodiments of the present disclosure and/or related embodiments include adding information from oxygenation saturation sensor 30. For example, sensor 30 can be a simple pulse oximeter sensor placed on a finger or ear lobe or nose, or a NIRS (near-infrared spectroscopy) device that monitors regional tissue oxygenation. Of note is that pulse oximetry measurements generally require pulsatile flow, so will be affected by low perfusion of a patient in cardiac arrest. Pulse oximetry measurements can also be affected by artifacts induced by the chest compression during CPR and inaccuracy with low oxygen saturation.

In accordance with certain exemplary embodiments of the present disclosure, (1) chest compressions may create enough of a "pulse" to allow pulse detection for measuring oxygen saturation, (2) the oxygen saturation signal can be distinguished from the compression artifacts (or be taken during a pause in compressions if needed), and (3) the patient's own oxygen reserve or oxygen administered through the course of the resuscitation treatment may allow for sufficient oxygen saturation.

Also in accordance with certain exemplary embodiments of the present disclosure, either alternatively or additionally, a measure of ETCO2 could be used in conjunction for CO2 production. This measurement is commonly done in resuscitations by placing respiratory sensor 40 between the patient's airway and ventilator. ETCO2 measurement can concurrently be used to assess the effectiveness of compressions, for example.

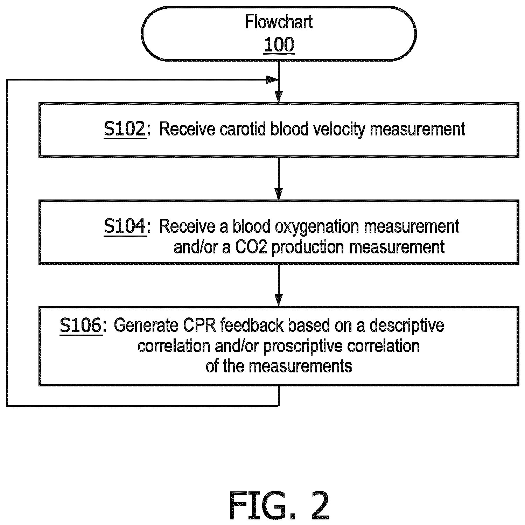

FIG. 2 illustrates a flowchart 100 representative of a CPR feedback method of the present disclosure. Referring to FIGS. 1 and 2, a stage S102 of flowchart 100 encompasses CPR feedback controller 60 receiving a carotid blood velocity measurement 21 from ultrasound sensor 20 during CPR of the patient, and a stage S104 of flowchart 100 encompasses CPR feedback controller 60 receiving a blood oxygenation measurement 31 from oxygenation saturation sensor 30 of the patient during CPR of the patient and/or a CO2 production measurement 41 from respiratory sensor 40 during the CPR of the patient.

A stage S106 of flowchart 100 encompasses CPR feedback controller 60 generating CPR feedback based on a descriptive correlation and/or a prescriptive correlation of the carotid blood velocity measurement 21 by the ultrasound sensor 20 and one or both of the blood oxygenation measurement 31 by the oxygenation saturation sensor 30 and a CO2 production measurement 41 by the respiratory sensor 40.

In practice, in accordance with certain exemplary embodiments of the present disclosure, the descriptive correlation of the measurements is implemented by machine learning of a relationship between the various measurements that is descriptive of an ongoing baseline CPR of the patient. In one such exemplary embodiment, for example, the descriptive correlation of the measurements is informative of an oxygen flow condition of the patient during the CPR of the patient, whereby the CPR feedback is informative and/or illustrative of the oxygen flow condition of the patient relative to a baseline oxygen flow of the patient and the CPR responder or the mechanical CPR device 90 may adjust the CPR technique as needed to converge the oxygen flow condition with the baseline oxygen flow.

In practice, in accordance with certain exemplary embodiments of the present disclosure,, the prescriptive correlation of the measurement is implemented by machine learning of a relationship between the various measurements that is prescriptive of a patient-specific CPR of the patient. In one such exemplary embodiment, for example, the prescriptive correlation of the measurements is informative of CPR parameter(s) associated with a baseline oxygen flow of the patient during the CPR of the patient, whereby the CPR feedback is informative and/or illustrative of adjustment(s) to CPR guideline(s) during the CPR of the patient based on the CPR parameter(s).

The following is more detailed explanation of a descriptive correlation and a prescriptive correlation.

**Descriptive Correlation.** Information on the patient's blood oxygenation or CO2 production as can be measured and monitored in accordance with certain exemplary embodiments of the present disclosure can inform controller 60 and care providers of the adequacy of the patient's ventilation leading to feedback on ventilation (e.g., during use of BVM, the bagging rate and volume may be adjusted), for example. Trending information can also be ascertained and help to identify the status and potential improvement of the patient. An exemplary novel and inventive aspect of this embodiment of the present disclosure is to correlate the O2 and CO2 measurements 31 and 41 with the carotid blood flow 21 to provide information on brain perfusion and respiratory measurements. By adjusting the CPR parameter(s) (e.g., compression rate, depth, duty cycle) based on (near) real-time feedback from such exemplary embodiments of present disclosure, the person(s) and/or automated/mechanical CPR device 90 administering CPR will typically be able to achieve an improved blood flow to the brain, for example.

The use of uCPR and other measures including oxygenation saturation sensor 30 to provide physiological CPR feedback can guide the responders to deliver patient-specific CPR that is responsive to the needs of a particular patient. Currently the CPR guidelines recommend a one-size-fit-all model on the depth and rate of compressions, e.g., 5-6 cm for depth and 100 - 120/min for rate. By leveraging the physiological feedback, responders may adjust their chest compression technique (while using the CPR guidelines as a baseline or starting point) towards achieving the desired blood flow for the physiological condition of the patient.

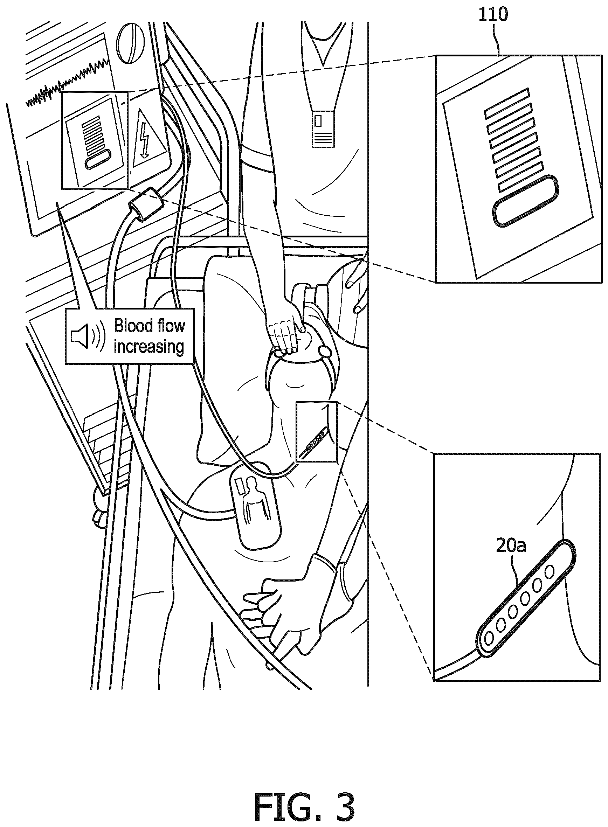

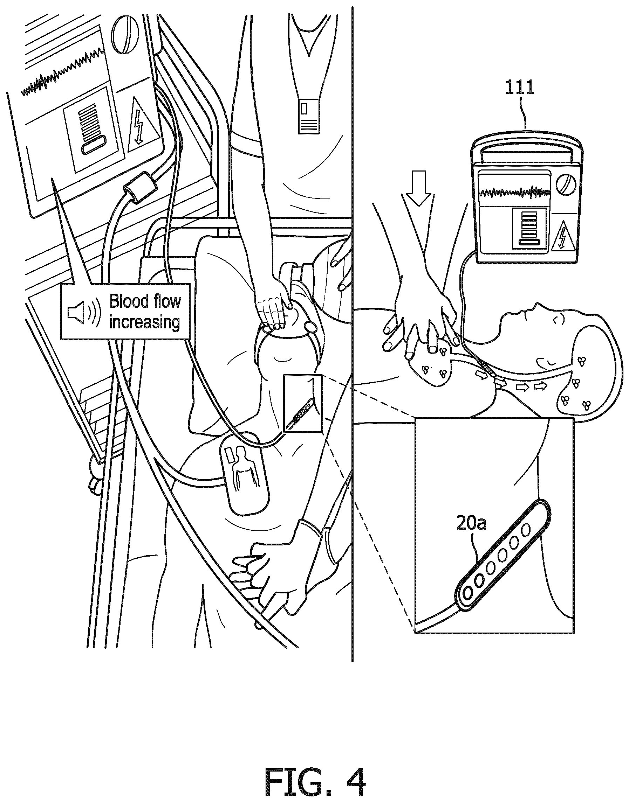

FIGS. 3-5 illustrates an embodiment of ultrasound sensor 20a having LEDs to indicate real-time blood velocity/flow with colors and different number of active LEDs. As shown in FIG. 3, a descriptive correlation of the measurements is informative of a "blood flow is increasing" condition that is illustrated via a CPR feedback 110 illustrative of the LED status of the ultrasound sensor 20, whereby the responder or mechanical CPR device 90 may maintain the CPR parameters as the blood flow increases. As shown in FIG. 4, a descriptive correlation of the measurements is informative of a "blood flow is decreasing" condition that is illustrated via a CPR feedback 111 illustrative of the LED status of the ultrasound sensor 20, whereby the responder or mechanical CPR device may adjust the CPR parameters to increase blood flow. As shown in FIG. 4, a descriptive correlation of the measurements is informative of a "pulse detected" condition that is illustrated via a CPR feedback 112 illustrative of the LED status of the ultrasound sensor 20, whereby or mechanical CPR device 90 may terminate the CPR.
For these examples, the display shows an infographic with a similar color scheme and visual representation.

**Prescriptive Correlation.** In accordance with certain exemplary embodiments of the present disclosure, the CPR feedback controller 70 may incorporate feedback devices that measure the mechanics of chest compression (e.g., Q-CPR which can measure and prompt depth, rate, and recoil. This will enable the person(s) and/or automated/mechanical device 90 performing CPR to know the mechanics of their compressions quantitatively, which may serve an additional CPR quality reference both at the beginning of and during the chest compression. For example, the CPR administrator (person(s) and/or automated/mechanical device 90) may start with rate and depth of compression that adheres to AHA Guidelines. The CPR administrator would know they/it are complying with Guidelines through a CPR coaching device (e.g., QCPR) that would provide feedback. The QCPR device would be integrated into controller 60 so that information such as rate and compression depth could be analyzed to further guide the person(s) administering CPR and/or automatically adjust the automated/mechanical device administering CPR. For example, if the CPR administrator is compressing at twice the Guideline's compression rate, the heart may not have time to fill adequately with blood. As a result, the uCPR device may detect very low carotid blood flow. The CPR administrator would be advised or instructed to slow down the rate of compressions. The same example can also work for compression depth, leaning and recoil, which can also be measured and used to provide feedback in conjunction with the flow measured through the carotid sensor, for example. When an optimal (peak) blood flow is/was measured through the uCPR device, the rate and depth of compression that is/was used to achieve this optimal flow can be noted/recorded/stored. If the blood flow began to decrease, the exemplary device could coach/advise/instruct the CPR administrator if they have deviated from their optimal rate/compression depth previously noted/recorded/stored, and/or provide instructions to an automated/mechanical CPR device 90 to adjust accordingly, to restore the optimal rate/compression depth.

Further, in accordance with certain exemplary embodiments of the present disclosure, it can also happen that as the patient's underlying physiology changes (e.g., ribs broken or blood accumulation in the pericardium) that the optimal rate/depth of compressions needs to change. Certain exemplary embodiments of the present disclosure described herein can detect these changes and trends and further coach/advise/instruct the CPR administrator (person(s) and/or mechanical 90) accordingly. The CPR administrator can use deeper compressions, a different rate, and/or adjust the position of contact on the chest (e.g., person hand, mechanical plunger), for example.

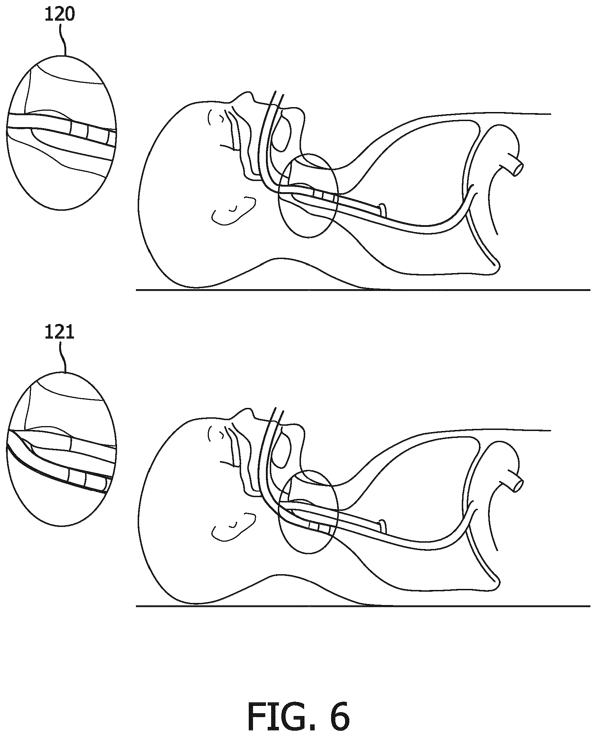

Additional exemplary embodiments in accordance with the present disclosure which are disclosed, described and taught herein include using the ultrasound sensor 20 in the form of a uCPR device to show and/or otherwise indicate if an intubation tube is properly placed in an esophagus vs a trachea. The exemplary uCPR device can be structured and configured to be a patch that can be placed and positioned on, near or around a person's neck and used for imaging the trachea and the esophagus at the level of the carotid vessels so that, in addition to measuring blood flow velocity, the exemplary uCPR device (e.g., patch) can be used to monitor and show, verify or otherwise indicate (e.g., via visual and/or audible alerts, prompts or instructions) a proper placement 120 or an improper placement 21 of the endotracheal tube placement and position as exemplary shown in FIG. 6.

Incorrect placement of the tube in the espohagus (or movement of the tube during patient treatment) can lead to air pushed into the stomach and gastric distension. This air pushes against internal organs including the heart and lungs causing damage in addition to lack of proper ventilation to the lungs. Incorrect placement occurs frequently and correct placement can be difficult to verify in the field and generally involves listening to lung and gastic sounds or use of visualization of the cords, CO2 monitoring and observation of symmetrical chest rise. These techniques can be susceptible to errors and misinterpretation, a problem overcome by certain exemplary embodiments of the present disclosure. Further, by using the exemplary uCPR device disclosed and described herein, the placement and positioning information is obtained in a continuous manner without additional equipment. Additionally, exemplary embodiments of CPR feedback controller 70 can use deep learning to automatically identify correct and/or incorrect placement based on image, e.g., to automatically trigger visual and/or audible alerts.

Another use of an exemplary patch uCPR device in accordance with the present disclosure is to identify full or partial blockages in the carotid artery. For example, this can be accomplished thru imagining of the carotid vessels or by analyzing the blood flow characteristics through the vessel as measured by the uCPR device. A blocked vessel will have a different flow velocity profile than an unblocked vessel. The exemplary CPR feedback controller can be structured and configured to automatically identify a blocked vessel from the image and generate/provide an alert or other indication.

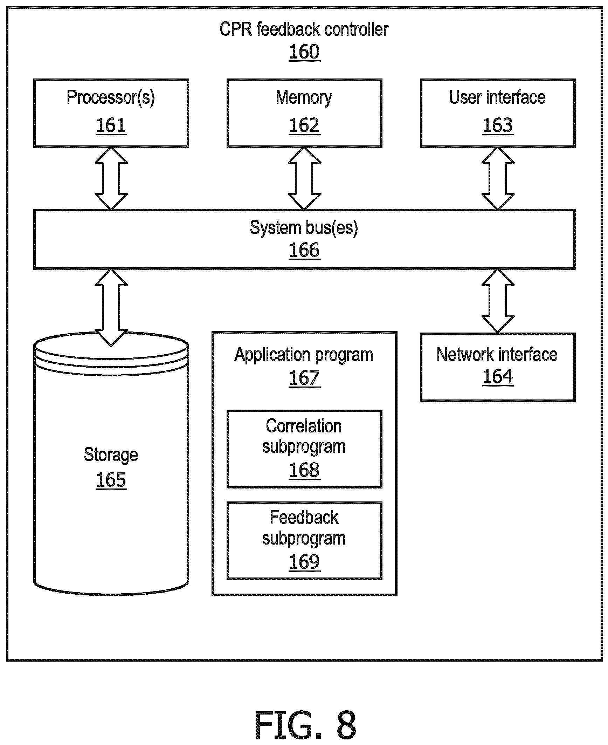

To facilitate a further understanding of the present disclosure, the following description of FIG. 8 teaches an exemplary embodiment of light indicator controller in accordance with the present disclosure. From the description of FIG. 8, those having ordinary skill in the art of the present disclosure will appreciate how to apply the present disclosure to make and use additional embodiments of a light indicator controller in accordance with the present disclosure.

Referring to FIG. 8, shown is an exemplary embodiment of CPR feedback controller 160 that includes one or more processor(s) 161, memory 162, a user interface 163, a network interface 164, and a storage 165 interconnected via one or more system bus(es) 166.

Each processor 161 can be any hardware device, as known in the art of the present disclosure or hereinafter conceived, capable of executing instructions stored in memory 162 or storage or otherwise processing data. In a non-limiting example, the processor(s) 161 can include a microprocessor, field programmable gate array (FPGA), application-specific integrated circuit (ASIC), or other similar devices.

The memory 162 can include various memories, as known in the art of the present disclosure or hereinafter conceived, including, but not limited to, L1, L2, or L3 cache or system memory. In a non-limiting example, the memory 162 can include static random access memory (SRAM), dynamic RAM (DRAM), flash memory, read only memory (ROM), or other similar memory devices.

The user interface 163 can include one or more devices, as known in the art of the present disclosure or hereinafter conceived, for enabling communication with a user such as an administrator. In a non-limiting example, the user interface can include a command line interface or graphical user interface that may be presented to a remote terminal via the network interface 164.

The network interface 164 can include one or more devices, as known in the art of the present disclosure or hereinafter conceived, for enabling communication other components of a medical device. In a non-limiting example, the network interface 164 can include a network interface card (NIC) configured to communicate according to the Ethernet protocol. Additionally, the network interface 164 may implement a TCP/IP stack for communication according to the TCP/IP protocols. Various alternative or additional hardware or configurations for the network interface 164 will be apparent.

The storage 165 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 165 may store instructions for execution by the processor(s) 161 or data upon with the processor(s) 161 may operate. For example, the storage 165 may store a base operating system for controlling various basic operations of the hardware.

The storage 165 can also store an application program in the form of executable software/firmware for implementing the various functions of the methods of FIG. 2 as previously described in the present disclosure. In one exemplary embodiment as shown, storage 165 also stores application program 167 including a correlation subprogram 168 a feedback subprogram 169 for implementing an embodiment of stage S106 of flowchart 100.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A cardiopulmonary resuscitation ("CPR") feedback system (10), comprising:
an ultrasound sensor (20) configured to measure carotid blood velocity of the patient during CPR of the patient;
at least one of:
an oxygenation saturation sensor (30) configured to measure blood oxygenation of the patient during CPR of the patient; or
a respiratory sensor (40) configured to measure CO2 production of the patient during CPR of the patient; and
a CPR feedback controller (60, 160) configured to generate CPR feedback (112) **characterized in that** the CPR feedback is based on at least one of a descriptive correlation or a prescriptive correlation of a carotid blood velocity measurement (21) by the ultrasound sensor and at least one of a blood oxygenation measurement (31) by the oxygenation saturation sensor or a CO2 production measurement (41) by the respiratory sensor.

2. The CPR feedback system of claim 1,
wherein the descriptive correlation of the carotid blood velocity measurement by the ultrasound sensor and at least one of the blood oxygenation measurement by the oxygenation saturation sensor or the CO2 production measurement by the respiratory sensor is informative of an oxygen flow condition of the patient during the CPR of the patient; and
wherein the CPR feedback is at least one of informative or illustrative of the oxygen flow condition of the patient relative to a baseline oxygen flow of the patient.

3. The CPR feedback system of claim 1,
wherein the prescriptive correlation of the carotid blood velocity measurement by the ultrasound sensor and at least one of the blood oxygenation measurement by the oxygenation saturation sensor or the CO2 production measurement by the respiratory sensor is informative of at least one CPR parameters associated with a baseline oxygen flow of the patient during the CPR of the patient; and
wherein the CPR feedback is at least one of informative or illustrative of at least one adjustment of a CPR guideline during the CPR of the patient based on the CPR parameters.

4. The CPR feedback system of claim 1,
wherein the ultrasound sensor is further configured to image a placement of an endotracheal tube within the patient at the level of a carotid vessel of the patient; and
wherein the CPR feedback controller is further configured to identify at least one of a correct placement or an incorrect placement of the endotracheal tube within the patient at the level of a carotid vessel of the patient based on the image.

5. The CPR feedback system of claim 1,
wherein the ultrasound sensor is further configured to image a carotid vessel of the patient; and
wherein the CPR feedback controller is further configured to identify any blockage within the carotid vessel of the patient based on the image.

6. A cardiopulmonary resuscitation ("CPR") feedback controller, comprising:
a non-transitory machine-readable storage medium encoded with instructions for execution by at least one processor, the non-transitory machine-readable storage medium including the instructions to:
receive (S102) a carotid blood velocity of a patient during CPR of the patient;
receive (S104) at least one of a blood oxygenation measurement of the patient or a CO2 production measurement of the patient during CPR of the patient; and
generate (S106) CPR feedback
**characterized in that** the CPR feedback is based on at least one of a descriptive correlation or a prescriptive correlation of a carotid blood velocity measurement and at least one of the blood oxygenation measurement or the CO2 production measurement.

7. The CPR feedback controller of claim 6,
wherein the descriptive correlation of the carotid blood velocity measurement and at least one of the blood oxygenation measurement or the CO2 production measurement is informative of an oxygen flow condition of the patient during the CPR of the patient; and
wherein the CPR feedback is at least one of informative or illustrative of the oxygen flow condition of the patient relative to a baseline oxygen flow of the patient.

8. The CPR feedback controller of claim 6,
wherein the prescriptive correlation of the carotid blood velocity measurement and at least one of the blood oxygenation measurement or the CO2 production measurement is informative of at least one CPR parameters associated with a baseline oxygen flow of the patient during the CPR of the patient; and
wherein the CPR feedback is at least one of informative or illustrative of at least one adjustment of a CPR guideline during the CPR of the patient based on the CPR parameters.

9. The CPR feedback controller of claim 6, wherein the non-transitory machine-readable storage medium further includes instructions to:
receive an image of a placement of an endotracheal tube within the patient at the level of a carotid vessel of the patient; and
identify at least one of a correct placement or an incorrect placement of the endotracheal tube within the patient at the level of a carotid vessel of the patient based on the image.

10. The CPR feedback controller of claim 6, wherein the non-transitory machine-readable storage medium further includes instructions to:
receive an image of a carotid vessel of the patient; and
identify any blockage within the carotid vessel of the patient based on the image.

11. A CPR feedback method, comprising:
measuring, by an ultrasound sensor, a carotid blood velocity of a patient during CPR of the patient;
at least one of:
measuring, by an oxygenation saturation sensor, a blood oxygenation of a patient during CPR of the patient; or
measuring, by a respiratory sensor, a CO2 production of a patient during CPR of the patient; and
generating, by a CPR feedback controller, CPR feedback
**characterized in that** the CPR feedback is based on at least one of a descriptive correlation or a prescriptive correlation of the carotid blood velocity measurement by the ultrasound sensor and at least one of the blood oxygenation measurement by the oxygenation saturation sensor or the CO2 production measurement by the respiratory sensor.

12. The CPR feedback method of claim 11,
wherein the descriptive correlation of the carotid blood velocity measurement by the ultrasound sensor and at least one of the blood oxygenation measurement by the oxygenation saturation sensor or the CO2 production measurement by the respiratory sensor is informative of an oxygen flow condition of the patient during the CPR of the patient; and
wherein the CPR feedback is at least one of informative or illustrative of the oxygen flow condition of the patient relative to a baseline oxygen flow of the patient.

13. The CPR feedback method of claim 11,
wherein the prescriptive correlation of the carotid blood velocity measurement by the ultrasound sensor and at least one of the blood oxygenation measurement by the oxygenation saturation sensor or the CO2 production measurement by the respiratory sensor is informative at least one CPR parameters associated with a baseline oxygen flow of the patient during the CPR of the patient; and
wherein the CPR feedback is at least one of informative or illustrative of at least one adjustment of a CPR guideline during the CPR of the patient based on the CPR parameters.

14. The CPR feedback method of claim 11, further comprising:
imaging, by the ultrasound sensor, a placement of an endotracheal tube within the patient at the level of a carotid vessel of the patient; and
identifying, by the CPR feedback controller, at least one of a correct placement (120) or an incorrect placement (121) of the endotracheal tube within the patient at the level of a carotid vessel of the patient based on the image.

15. The CPR feedback method of claim 11, further comprising:
imaging, by the ultrasound sensor, a carotid vessel of the patient; and
identifying, by the CPR feedback controller, any blockage within the carotid vessel of the patient based on the image.

## Patentansprüche

1. Rückkopplungssystem (10) zur Herz-Lungen-Wiederbelebung ("CPR"), umfassend:
einen Ultraschallsensor (20), der zum Messen der Carotis-Blutgeschwindigkeit des Patienten während der CPR des Patienten konfiguriert ist;
mindestens eines von:
einem Sauerstoffsättigungssensor (30), der zum Messen der Blutsauerstoffsättigung des Patienten während der CPR des Patienten konfiguriert ist; oder
einem Atmungssensor (40), der zum Messen der CO2-Produktion des Patienten während der CPR des Patienten konfiguriert ist; und
einer CPR-Rückkopplungssteuereinheit (60, 160), die konfiguriert ist, um CPR-Rückkopplung (112) zu erzeugen, **dadurch gekennzeichnet, dass** die CPR-Rückkopplung auf mindestens einer von einer deskriptiven Korrelation oder einer präskriptiven Korrelation einer Carotis-Blutgeschwindigkeitsmessung (21) mittels des Ultraschallsensors und mindestens einer von einer Blutsauerstoffsättigungsmessung (31) mittels des Sauerstoffsättigungssensors oder einer CO2-Produktionsmessung (41) mittels des Atmungssensors basiert.

2. CPR-Rückkopplungssystem nach Anspruch 1,
wobei die deskriptive Korrelation der Carotis-Blutgeschwindigkeitsmessung mittels des Ultraschallsensors und mindestens einer der Blutsauerstoffsättigungsmessung mittels des Sauerstoffsättigungssensors oder der CO2-Produktionsmessung mittels des Atmungssensors über einen Sauerstoffflusszustand des Patienten während der CPR des Patienten informiert; und
wobei die CPR-Rückkopplung mindestens entweder informativ oder illustrativ für den Sauerstoffflusszustand des Patienten relativ zu einem Basislinien-Sauerstofffluss des Patienten ist.

3. CPR-Rückkopplungssystem nach Anspruch 1,
wobei die präskriptive Korrelation der Carotis-Blutgeschwindigkeitsmessung mittels des Ultraschallsensors und mindestens einer der Blutsauerstoffsättigungsmessung mittels des Sauerstoffsättigungssensors oder der CO2-Produktion mittels des Atmungssensors über mindestens einen CPR-Parameter informiert, der einem Basislinien-Sauerstofffluss des Patienten während der CPR des Patienten zugeordnet ist; und
wobei die CPR-Rückkopplung mindestens entweder informativ oder illustrativ für mindestens eine Anpassung einer CPR-Richtlinie während der CPR des Patienten auf der Basis der CPR-Parameter ist.

4. CPR-Rückkopplungssystem nach Anspruch 1,
wobei der Ultraschallsensor weiter konfiguriert ist, um eine Platzierung eines Endotrachealtubus innerhalb des Patienten auf Höhe eines Carotis-Gefäßes des Patienten abzubilden; und
wobei die CPR-Rückkopplungssteuereinheit weiter konfiguriert ist, um auf der Basis des Bildes mindestens eine von einer korrekten Platzierung oder einer fehlerhaften Platzierung des Endotrachealtubus innerhalb des Patienten auf Höhe eines Carotis-Gefäßes des Patienten zu identifizieren.

5. CPR-Rückkopplungssystem nach Anspruch 1,
wobei der Ultraschallsensor weiter konfiguriert ist, um ein Carotis-Gefäß des Patienten abzubilden; und
wobei der CPR-Rückkopplungssteuereinheit weiter konfiguriert ist, um auf der Basis des Bildes jede Blockade innerhalb des Carotis-Gefäßes des Patienten zu identifizieren.

6. Rückkopplungssteuereinheit zur Herz-Lungen-Wiederbelebung ("CPR"), umfassend:
ein nicht transitorisches, maschinenlesbares Speichermedium, das mit Anweisungen zur Ausführung mittels mindestens eines Prozessors codiert ist, wobei das nicht transitorische, maschinenlesbare Speichermedium die Anweisungen einschließt zum:
Empfangen (S102) einer Carotis-Blutgeschwindigkeit eines Patienten während der CPR des Patienten;
Empfangen (S104) von mindestens einer von einer Blutsauerstoffsättigungsmessung des Patienten oder einer CO2-Produktionsmessung des Patienten während der CPR des Patienten; und
Erzeugen (S106) von CPR-Rückkopplung,
**dadurch gekennzeichnet, dass** die CPR-Rückkopplung auf mindestens einer von einer deskriptiven Korrelation oder einer präskriptiven Korrelation einer Carotis-Blutgeschwindigkeitsmessung und mindestens einer von der Blutsauerstoffsättigungsmessung oder der CO2-Produktionsmessung basiert.

7. CPR-Rückkopplungssteuereinheit nach Anspruch 6,
wobei die deskriptive Korrelation der Carotis-Blutgeschwindigkeitsmessung und mindestens eine der Blutsauerstoffsättigungsmessung oder der CO2-Produktionsmessung über einen Sauerstoffflusszustand des Patienten während der CPR des Patienten informiert; und
wobei die CPR-Rückkopplung mindestens entweder informativ oder illustrativ für den Sauerstoffflusszustand des Patienten relativ zu einem Basislinien-Sauerstofffluss des Patienten ist.

8. CPR-Rückkopplungssteuereinheit nach Anspruch 6,
wobei die präskriptive Korrelation der Carotis-Blutgeschwindigkeitsmessung und mindestens eine der Blutsauerstoffsättigungsmessung oder der CO2-Produktionsmessung über mindestens einen CPR-Parameter informiert, der einem Basislinien-Sauerstofffluss des Patienten während der CPR des Patienten zugeordnet ist; und
wobei die CPR-Rückkopplung mindestens entweder informativ oder illustrativ für mindestens eine Anpassung einer CPR-Richtlinie während der CPR des Patienten auf der Basis der CPR-Parameter ist.

9. CPR-Rückkopplungssteuereinheit nach Anspruch 6, wobei das nicht transitorische, maschinenlesbare Speichermedium weiter Anweisungen einschließt zum:
Empfangen eines Bildes einer Platzierung eines Endotrachealtubus innerhalb des Patienten auf Höhe eines Carotis-Gefäßes des Patienten; und
Identifizieren auf der Basis des Bildes mindestens einer von einer korrekten Platzierung oder einer fehlerhaften Platzierung des Endotrachealtubus innerhalb des Patienten auf Höhe eines Carotis-Gefäßes des Patienten.

10. CPR-Rückkopplungssteuereinheit nach Anspruch 6, wobei das nicht transitorische, maschinenlesbare Speichermedium weiter Anweisungen einschließt zum:
Empfangen eines Bildes eines Carotis-Gefäßes des Patienten; und
Identifizieren jeder Blockade innerhalb des Carotis-Gefäßes des Patienten auf der Basis des Bildes.

11. CPR-Rückkopplungsverfahren, umfassend:
Messen der Carotis-Blutgeschwindigkeit eines Patienten während der CPR des Patienten mittels eines Ultraschallsensors;
mindestens eines von:
Messen einer Blutsauerstoffsättigung eines Patienten während der CPR des Patienten mittels eines Sauerstoffsättigungssensors; oder
Messen einer CO2-Produktion eines Patienten während der CPR des Patienten mittels eines Atmungssensors; und
Erzeugen von CPR-Rückkopplung mittels eines CPR-Rückkopplungssteuereinheit,
**dadurch gekennzeichnet, dass** die CPR-Rückkopplung auf mindestens einer von einer deskriptiven Korrelation oder einer präskriptiven Korrelation der Carotis-Blutgeschwindigkeitsmessung mittels des Ultraschallsensors und mindestens einer der Blutsauerstoffsättigungsmessung mittels des Sauerstoffsättigungssensors oder der CO2-Produktionsmessung mittels des Atmungssensors basiert.

12. CPR-Rückkopplungsverfahren nach Anspruch 11,
wobei die deskriptive Korrelation der Carotis-Blutgeschwindigkeitsmessung mittels des Ultraschallsensors und mindestens einer der Blutsauerstoffsättigungsmessung mittels des Sauerstoffsättigungssensors oder der CO2-Produktionsmessung mittels des Atmungssensors über einen Sauerstoffflusszustand des Patienten während der CPR des Patienten informiert; und
wobei die CPR-Rückkopplung mindestens entweder informativ oder illustrativ für den Sauerstoffflusszustand des Patienten relativ zu einem Basislinien-Sauerstofffluss des Patienten ist.

13. CPR-Rückkopplungsverfahren nach Anspruch 11,
wobei die präskriptive Korrelation der Carotis-Blutgeschwindigkeitsmessung mittels des Ultraschallsensors und mindestens einer der Blutsauerstoffsättigungsmessung mittels des Sauerstoffsättigungssensors oder der CO2-Produktion mittels des Atmungssensors über mindestens einen CPR-Parameter informiert, der einem Basislinien-Sauerstofffluss des Patienten während der CPR des Patienten zugeordnet ist; und
wobei die CPR-Rückkopplung mindestens entweder informativ oder illustrativ für mindestens eine Anpassung einer CPR-Richtlinie während der CPR des Patienten auf der Basis der CPR-Parameter ist.

14. CPR-Rückkopplungsverfahren nach Anspruch 11, weiter umfassend:
Abbilden einer Platzierung eines Endotrachealtubus innerhalb des Patienten auf Höhe eines Carotis-Gefäßes des Patienten mittels des Ultraschallsensors; und
Identifizieren auf der Basis des Bildes mindestens einer von einer korrekten Platzierung (120) oder einer fehlerhaften Platzierung (121) des Endotrachealtubus innerhalb des Patienten auf Höhe eines Carotis-Gefäßes des Patienten mittels der CPR-Rückkopplungssteuereinheit.

15. CPR-Rückkopplungsverfahren nach Anspruch 11, weiter umfassend:
Abbilden eines Carotis-Gefäßes des Patienten mittels des Ultraschallsensors; und
Identifizieren jeder Blockade innerhalb des Carotis-Gefäßes des Patienten auf der Basis des Bildes mittels der CPR-Rückkopplungssteuereinheit.

## Revendications

1. Système de rétroaction de réanimation cardio-pulmonaire (« CPR ») (10), comprenant :
un capteur à ultrasons (20) configuré pour mesurer la vitesse du sang de la carotide du patient pendant la CPR du patient ;
au moins un parmi :
un capteur de saturation en oxygénation (30) configuré pour mesurer l'oxygénation du sang du patient pendant la CPR du patient ; ou
un capteur respiratoire (40) configuré pour mesurer la production de CO2 du patient pendant la CPR du patient ; et
un dispositif de commande de rétroaction de CPR (60, 160) configuré pour générer une rétroaction de CPR (112) **caractérisé en ce que** la rétroaction de CPR est basée sur au moins une parmi une corrélation descriptive ou une corrélation prescriptive d'une mesure de la vitesse du sang de la carotide (21) par le capteur à ultrasons et d'au moins une parmi une mesure de l'oxygénation du sang (31) par le capteur de saturation en oxygénation ou une mesure de la production de CO2 (41) par le capteur respiratoire.

2. Système de rétroaction de CPR selon la revendication 1,
dans lequel la corrélation descriptive de la mesure de la vitesse du sang de la carotide par le capteur à ultrasons et d'au moins une parmi la mesure de l'oxygénation du sang par le capteur de saturation en oxygénation ou la mesure de la production de CO2 par le capteur respiratoire fournit des informations sur l'état du débit d'oxygène du patient pendant la CPR du patient ; et
dans lequel la rétroaction de CPR est au moins une parmi informative ou illustrative de l'état du débit d'oxygène du patient par rapport à un débit d'oxygène initial du patient.

3. Système de rétroaction de CPR selon la revendication 1,
dans lequel la corrélation prescriptive de la mesure de la vitesse du sang de la carotide par le capteur à ultrasons et d'au moins une parmi la mesure de l'oxygénation du sang par le capteur de saturation en oxygénation ou la mesure de la production de CO2 par le capteur respiratoire fournit des informations sur au moins un paramètre de CPR associé à un débit d'oxygène initial du patient pendant la CPR du patient ; et
dans lequel la rétroaction de CPR est au moins une parmi informative ou illustrative d'au moins un ajustement d'une directive en matière de CPR pendant la CPR du patient sur la base des paramètres de CPR.

4. Système de rétroaction de CPR selon la revendication 1,
dans lequel le capteur à ultrasons est configuré en outre pour imager un placement d'un tube endotrachéal à l'intérieur du patient au niveau d'un vaisseau carotidien du patient ; et
dans lequel le dispositif de commande de rétroaction de CPR est configuré en outre pour identifier au moins un parmi un placement correct ou un placement incorrect du tube endotrachéal à l'intérieur du patient au niveau d'un vaisseau carotidien du patient sur la base de l'image.

5. Système de rétroaction de CPR selon la revendication 1,
dans lequel le capteur à ultrasons est configuré en outre pour imager un vaisseau carotidien du patient ; et
dans lequel le dispositif de commande de rétroaction de CPR est configuré en outre pour identifier tout blocage dans le vaisseau carotidien du patient sur la base de l'image.

6. Dispositif de commande de rétroaction de réanimation cardio-pulmonaire (« CPR »), comprenant :
un support de stockage non transitoire lisible par machine codé avec des instructions destinées à être exécutées par au moins un processeur, le support de stockage non transitoire lisible par machine incluant les instructions pour :
recevoir (S102) une vitesse du sang de la carotide d'un patient pendant la CPR du patient ;
recevoir (S104) au moins une parmi une mesure de l'oxygénation du sang du patient ou une mesure de la production de CO2 du patient pendant la CPR du patient ; et
générer (S106) une rétroaction de CPR
**caractérisé en ce que** la rétroaction de CPR est basée sur au moins une parmi une corrélation descriptive ou une corrélation prescriptive d'une mesure de la vitesse du sang de la carotide et d'au moins une parmi la mesure de l'oxygénation du sang ou la mesure de la production de CO2.

7. Dispositif de commande de rétroaction de CPR selon la revendication 6,
dans lequel la corrélation descriptive de la mesure de la vitesse du sang de la carotide et d'au moins une parmi la mesure de l'oxygénation du sang ou la mesure de la production de CO2 fournit des informations sur l'état du débit d'oxygène du patient pendant la CPR du patient ; et
dans lequel la rétroaction de CPR est au moins une parmi informative ou illustrative de l'état du débit d'oxygène du patient par rapport à un débit d'oxygène initial du patient.

8. Dispositif de commande de rétroaction de CPR selon la revendication 6,
dans lequel la corrélation prescriptive de la mesure de la vitesse du sang de la carotide et d'au moins une parmi la mesure de l'oxygénation du sang ou la mesure de la production de CO2 fournit des informations sur au moins un paramètre de CPR associé à un débit d'oxygène initial du patient pendant la CPR du patient ; et
dans lequel la rétroaction de CPR est au moins une parmi informative ou illustrative d'au moins un ajustement d'une directive en matière de CPR pendant la CPR du patient sur la base des paramètres de CPR.

9. Dispositif de commande de rétroaction de CPR selon la revendication 6, dans lequel le support de stockage non transitoire lisible par machine inclut en outre des instructions pour :
recevoir une image d'un placement d'un tube endotrachéal à l'intérieur du patient au niveau d'un vaisseau carotidien du patient ; et
identifier au moins un parmi un placement correct ou un placement incorrect du tube endotrachéal à l'intérieur du patient au niveau d'un vaisseau carotidien du patient sur la base de l'image.

10. Dispositif de commande de rétroaction de CPR selon la revendication 6, dans lequel le support de stockage non transitoire lisible par machine inclut en outre des instructions pour :
recevoir une image d'un vaisseau carotidien du patient ; et
identifier tout blocage dans le vaisseau carotidien du patient sur la base de l'image.

11. Procédé de rétroaction de CPR, comprenant :
la mesure, par un capteur à ultrasons, d'une vitesse du sang de la carotide d'un patient pendant la CPR du patient ;
au moins une parmi :
la mesure, par un capteur de saturation en oxygénation, d'une oxygénation du sang d'un patient pendant la CPR du patient ; ou
la mesure, par un capteur respiratoire, d'une production de CO2 d'un patient pendant la CPR du patient ; et
la génération, par un dispositif de commande de rétroaction de CPR, d'une rétroaction de CPR
**caractérisé en ce que** la rétroaction de CPR est basée sur au moins une parmi une corrélation descriptive ou une corrélation prescriptive de la mesure de la vitesse du sang de la carotide par le capteur à ultrasons et d'au moins une parmi la mesure de l'oxygénation du sang par le capteur de saturation en oxygénation ou la mesure de la production de CO2 par le capteur respiratoire.

12. Procédé de rétroaction de CPR selon la revendication 11,
dans lequel la corrélation descriptive de la mesure de la vitesse du sang de la carotide par le capteur à ultrasons et d'au moins une parmi la mesure de l'oxygénation du sang par le capteur de saturation en oxygénation ou la mesure de la production de CO2 par le capteur respiratoire fournit des informations sur l'état du débit d'oxygène du patient pendant la CPR du patient ; et
dans lequel la rétroaction de CPR est au moins une parmi informative ou illustrative de l'état du débit d'oxygène du patient par rapport à un débit d'oxygène initial du patient.

13. Procédé de rétroaction de CPR selon la revendication 11,
dans lequel la corrélation prescriptive de la mesure de la vitesse du sang de la carotide par le capteur à ultrasons et d'au moins une parmi la mesure de l'oxygénation du sang par le capteur de saturation en oxygénation ou la mesure de la production de CO2 par le capteur respiratoire fournit des informations sur au moins un paramètre de CPR associé à un débit d'oxygène initial du patient pendant la CPR du patient ; et
dans lequel la rétroaction de CPR est au moins une parmi informative ou illustrative d'au moins un ajustement d'une directive en matière de CPR pendant la CPR du patient sur la base des paramètres de CPR.

14. Procédé de rétroaction de CPR selon la revendication 11, comprenant en outre :
l'imagerie, par le capteur à ultrasons, d'un placement d'un tube endotrachéal à l'intérieur du patient au niveau d'un vaisseau carotidien du patient ; et
l'identification, par le dispositif de commande de rétroaction de CPR, d'au moins un parmi un placement correct (120) ou un placement incorrect (121) du tube endotrachéal à l'intérieur du patient au niveau d'un vaisseau carotidien du patient sur la base de l'image.

15. Procédé de rétroaction de CPR selon la revendication 11, comprenant en outre :
l'imagerie, par le capteur à ultrasons, d'un vaisseau carotidien du patient ; et
l'identification, par le dispositif de commande de rétroaction de CPR, de tout blocage dans le vaisseau carotidien du patient sur la base de l'image.
